(19) **Europäisches Patentamt / European Patent Office / Office européen des brevets**

(11) **EP 1 150 604 B1**

(12) **EUROPEAN PATENT SPECIFICATION**

(45) Date of publication and mention
of the grant of the patent:
**22.08.2007 Bulletin 2007/34**

(51) Int Cl.:
*A61B 5/0285* (2006.01)   *A61B 5/0225* (2006.01)

(21) Application number: **00903948.8**

(86) International application number:
**PCT/IL2000/000089**

(22) Date of filing: **11.02.2000**

(87) International publication number:
**WO 2000/047110 (17.08.2000 Gazette 2000/33)**

(54) **METHOD AND DEVICE FOR CONTINUOUS ANALYSIS OF CARDIOVASCULAR ACTIVITY OF A SUBJECT**

VERFAHREN UND VORRICHTUNG ZUR KONTINUIERLICHEN ANALYSE CARDIOVASKULÄRER AKTIVITÄT IN EINEM SUBJEKT

PROCEDE ET DISPOSITIF D'ANALYSE EN CONTINU D'UNE ACTIVITE CARDIO-VASCULAIRE D'UN SUJET

(84) Designated Contracting States:
**AT BE CH CY DE DK ES FI FR GB GR IE IT LI LU MC NL PT SE**

(30) Priority: **11.02.1999 IL 12848299**

(43) Date of publication of application:
**07.11.2001 Bulletin 2001/45**

(73) Proprietor: **Ultrasis International (1993) Ltd
91482 Jerusalem (IL)**

(72) Inventors:
• **SHUSTERMAN, Vladimir
Pittsburgh, PA 1521 (US)**

• **ROYTVARF, Alex
75257 Rishon Lezion (IL)**
• **ORBACH, Tuvi,
Ultramind International Ltd
91482 Jerusalem (IL)**

(74) Representative: **Gallafent, Richard John
GALLAFENT & CO.,
27 Britton Street,
London EC1M 5UD (GB)**

(56) References cited:
**EP-A- 0 443 267       EP-A- 0 824 009
WO-A-98/25516       US-A- 5 309 916**

**Description**

**FIELD OF THE INVENTION**

The invention is in the field of medical diagnostic devices and more specifically devices for analyzing cardiovascular activity of a subject.

**GLOSSARY**

There follows a glossary of terms used herein, some of which are standard, others having been coined, together with their abbreviations.
*Plethysmograph (PG)* - An instrument for measuring blood flow.
*Pulse Transit Time (PTT)* - The elapsed time between the arrival of a pulse pressure peak at two points in the arterial system, or the elapsed time between a particular point in the ECG signal and the arrival of the consequent pulse wave at a particular point in the arterial system.
*Cardiac output (CO) -* The blood volume pumped into the aorta by the heart per minute.
*Vascular compliance (VCL) -* The ratio of the change in the blood vessel volume to the change in pressure.
*AREA -* The area under the peak of a plethysmograph signal.
*Peak Amplitude (PA)* - The amplitude of the peak of a plethysmograph signal.
*Systolic Pressure (SP) -* The blood pressure during the contraction phase of the cardiac cycle.
*Diastolic Pressure (DP) -* The blood pressure during the relaxation period of the cardiac cycle.

**BACKGROUND OF THE INVENTION**

Continuous, non-invasive monitoring of blood pressure and vascular parameters is important, for example, in people for whom abnormally high or low blood pressure poses a major threat to their health. Several approaches have been developed for noninvasive, continuous, blood pressure monitoring. For example, U.S. Patent 4,475,554 discloses a device which determines blood pressure from oscillometric measurements. These devices utilize an inflatable cuff that can be placed on an arm above the elbow or on a finger. The cuff is inflated to equilibrate with the internal pressure in the underlying digital vessels. As the blood pressure in the digital arteries fluctuates, the cuff pressure is adjusted by a feedback control mechanism so as to balance the blood pressure. The blood pressure at any moment is considered to be proportional to the cuff pressure. This assumes that the elasticity and tone of the digital arteries remains constant over time while in fact it is extremely variable. For this reason, these devices are not practical for prolonged blood pressure monitoring. Moreover, the constant cuff pressure makes these cuffs uncomfortable for the patient and often causes problems in the peripheral blood circulation. These oscillometric devices are therefore rarely used for continuous blood pressure monitoring.
Several studies have attempted to estimate systolic and diastolic pressure by analyzing only the plethysmograph (PG) signal. These methods, however, employ high order derivatives and therefore require a signal with extremely low noise that is practically unattainable due to the subject's movements. Moreover, these methods cannot be used for real time blood pressure measurements since the data must be averaged over several minutes.
US-A-4869262, US-A-4807638 and US-A-5709212 disclose devices that calculate blood pressure only from pulse transient time (PTT). The reliability and reproducibility of blood pressure measurements determined solely from PTT, however, is not great enough to allow accurate blood pressure measurements.
Another approach to non-invasive monitoring of blood pressure is disclosed in European Patent Application EP-A-443267. This method uses both the PG signal and a PTT for the calculation of the systolic and diastolic pressures. The PG signal must first be normalised in each cardiac cycle by dividing the AC signal by the DC signal assuming that the variation in vascular tone and elasticity is slower than that in the heart rate. This normalisation procedure is empirical and inaccurate. Moreover, the equations used for calculating the systolic and diastolic pressures are also empirical and thus inaccurate in many cases.
US-A-5309916 discloses a device and method for determining continuously and non-invasively systolic and diastolic blood pressure as well as Young's modulus of an artery by obtaining continuously and non-invasively the subject's blood flow velocity and the propagation speed of the subject's pulse wave. This known technique is based on a number of empirical assumptions and requires a calibration process.
It has long been known that changes in cardiac output and other vascular characteristics (compliance, resistance and Young modulus) affect blood pressure. Different physiological processes govern blood pressure changes of different origins and a different medical treatment is required for the same change in blood pressure when it arises from different origins. Determining the cause of a change in blood pressure is therefore crucial for successful treatment. However, none of the prior art devices and methods discloses means for the non-evasive monitoring of these factors. Moreover, all of the prior art devices and methods ignore the effects of these factors on blood pressure.

There is accordingly a need in the art for a method and device for the non-invasive, continuous monitoring of blood pressure, cardiac output and other vascular characteristics in which the disadvantages of the aforementioned prior art methods are substantially reduced or eliminated.

In the context of the present invention, two explicitly described, calculable or measurable variables are considered equivalent to each other when the two variables are proportional to each other.

In the following description and set of claims, $\kappa$ will be used to denote the ratio of the blood flow velocity to the propagation speed of the pressure pulse wave in an individual.

The invention is based on the novel and non-obvious finding that diastolic and systolic blood pressures determined from calculations involving $\kappa$ are more accurate than those obtained by prior art methods where $\kappa$ is not used.

The invention thus comprises a method and a device for the continuous and non-invasive measurement of $\kappa$ as set out in the main claims. In a preferred embodiment of the invention, $\kappa$ is obtained from a PG signal and PTT of a subject. In a most preferred embodiment, $\kappa$ is obtained from a PG signal and PTT of a subject according to the theory of waves of strong discontinuities as described, for example, in Landau, L.D. and Lifshitz E.M., Statistical Physics, Pergamon Press, 1979; Landau, L.D. and Lifshitz E.M., Fluid Mechanics, Pergamon Press, 1987; and Landau, L.D. and Lifshitz E.M., Theory of Elasticity, Pergamon Press, 1986, and Kaplan D and Glass, Understanding Non-Linear Dynamics, Springer-Verlag, N.Y., 1995.

In a yet more preferred embodiment, $\kappa$ is given by:

$$\kappa = 1/(1/(PEAK \cdot v) + 1),$$

where v is the propagation speed of the pulse wave (the pulse wave velocity) which is inversely proportional to PTT, and

$$PEAK = k_1 \cdot PTT \cdot PA + k_2 \cdot AREA,$$

where PA and AREA are respectively the amplitude and area of the pulse wave obtained from the PG signal, and $k_1$ and $k_2$ are two empirically obtained constants.

In another preferred embodiment, $\kappa$ is given by:

$$\kappa = \frac{1}{\left(\left(\frac{1}{PA}\right) + 1\right)}$$

Slow (0.01-0.05 Hz) fluctuations in vascular radius (vasomotor tone) can optionally be filtered out from the PG signal in order to increase the accuracy of the $\kappa$ measurement. This can be carried out, for example, by replacing PEAK in the definition of $\kappa$ with PEAK/(slow component of PEAK)$^2$. The slow component of PEAK can be obtained, for example, by low-pass filtering of the pulse wave. Other methods for obtaining $\kappa$ continuously and non-invasively are also contemplated within the scope of the invention.

Means for obtaining the PG signal of a subject continuously and non-invasively is known in the art and may, for example, be a photo-PG sensor. Other methods for measuring pressure waves in a blood vessel are also contemplated within the scope of the invention. This includes, but is not limited to, use of several photo PG devices, impedance PG devices, piezoelectric, ultrasound, laser, or other types of sensors.

Means for the continuous and non-invasive determination of PTT is known in the art and may comprise, for example, an electrocardiograph monitor and a PG sensor. The PTT in this case is the time lapse between a particular point in the ECG wave, for example the R peak, and the arrival of the corresponding pressure wave at the PG sensor. Other means for measuring PTT comprise, for example, a pair of PG sensors that are attached to the skin along the same arterial vessel and separated from one another. In this case, the PPT is the time lapse between the arrival of a pressure wave at the two locations.

The invention thus further provides for a device for processing $\kappa$ in real time so as to obtain a continuous and non-invasive measurement of systolic and diastolic blood pressures.

Still further, the invention provides for a device for processing $\kappa$ in real time so as to obtain a continuous and non-invasive measurement of Young modulus, vascular resistance, cardiac output, and vascular compliance. The prior art does not disclose methods for obtaining these parameters.

The measurements provided by the invention of the diastolic and systolic blood pressures, Young modulus, vascular resistance, cardiac output, and vascular compliance are more robust and less sensitive to external noises, changes in body position, and sensor placement than measurements provided by prior art devices.

The invention further provides for a device for processing $\kappa$ in real time so as to continuously and non-invasively obtain indices for indicating a change in the blood pressure in a subject due to a change in cardiac output or a change in vascular compliance. Since different physiological processes govern blood pressure changes of different origins and a different medical treatment is required for the same change in blood pressure when it arises from different origins, the present invention provides means for determining the appropriate treatment.

In a preferred embodiment, $\kappa$ is processed in real time so as to obtain the aforementioned parameters according to the theory of waves of strong discontinuities. In a most preferred embodiment, the aforementioned parameters are obtained using the following algorithmic expressions:

**Systolic Pressure (SP)**

Method 1

$$SP = \rho v^2 \Phi(\kappa, \gamma),$$

where $\rho$ is the blood density, $\gamma$ is the thermodynamic Poisson exponent of the blood, and

$$\Phi = \frac{\sqrt{2\kappa(\gamma - 1)^2 + 4 \cdot (\gamma - 1) + 1} - 1}{2(\gamma - 1)}$$

Method 2

$$SP = (\log v^2)/\alpha + 2\rho \, v^2 \kappa/3 + \lambda,$$

where $\lambda = (\log(2\rho R/E_0 h))/\alpha$, where R is the radius of the artery, h is the thickness of the arterial wall, $E_0$ is Young modulus referred to zero pressure, and $\alpha$ is an empirically obtained constant.

Method 3

$$SP = (\log v^2/(1 - \varepsilon H^2))/\alpha + 2\rho v^2\kappa/3 + \lambda,$$

where $\varepsilon$ is an empirically obtained constant and H is the heart rate.

Method 4

$$SP = [(\log v^2)/\alpha + \lambda]/(1 - \kappa).$$

Method 5

$$SP = [(\log v^2/(1 - \varepsilon H^2))/\alpha + \lambda]/(1 - \kappa).$$

**Diastolic Pressure (DP)**

$$DP = SP - \rho v^2\kappa,$$

**Young Modulus**

Method 1

$$E = (2R/h)\,(SP\text{-}DP)/\kappa$$

Method 2

$$E = (2R/h)\,SP/\Phi\,(\kappa,\gamma)$$

Method 3

$$E = (2R/h)\ \rho\ \exp[(-\lambda+MP)\alpha]$$

where MP is the mean pressure, MP = (SP + 2·DP)/3, where SP or DP is obtained using an algorithmic expression involving $\kappa$.

Method 4

$$E = (2R/h)\cdot\rho\cdot\exp\ ((-\lambda+SP\cdot(1-\kappa))\alpha)$$

**Cardiac Output (CO)**

$$CO = PEAK\ \cdot\ \{\ v\ \cdot\ [1 + SP/(2\rho\cdot v^2)]\}^2$$

where SP is obtained using an algorithmic expression involving $\kappa$, and the slow component of PEAK has been filtered out as described above.

**Vascular Resistance (VR)**

$$VR = (SP - DP)/CO.$$

where any one or more of SP, DP, and CO are obtained using an algorithmic expression involving $\kappa$.

**Vascular Compliance (VC)**

$$VC = PEAK/(SP - DP).$$

where any one or more of SP, and DP are obtained from a calculation involving $\kappa$. Other methods for obtaining vascular compliance from $\kappa$ are also contemplated within the scope of the invention.

**The Effect of VC, VR and CO on Blood Pressure**

The relative contribution of CO to an observed change in SP is given by a parameter INDEX 1 defined by

$$\mathrm{INDEX1} = \partial SP/\partial CO - \partial SP/\partial VC$$

where any one or more of the parameters SP, CO, and VC are obtained from a calculation involving $\kappa$. An increase in INDEX1 over time is indicative of a change in SP primarily due to changes in cardiac output (CO). A decrease in INDEX1 over time is indicative of a changes in SP primarily due to a change in vascular compliance (VC).

The relative contribution of VR and CO to an observed change in SP is given by a parameter INDEX2 defined by

$$\mathrm{INDEX2} = \partial SP/\partial CO - \partial SP/\partial VR$$

where any one or more of the parameters SP, CO, and VR are obtained from a calculation involving $\kappa$. An increase in INDEX2 over time is indicative of a change in SP primarily due to changes in cardiac output (CO). A decrease in INDEX2 over time is indicative of a change in SP and DP primarily due to a change in vascular resistance (VR).

The invention will now be described by way of example only with reference to the accompanying non-limiting drawings in which:

> Fig. 1 shows one embodiment using a device of the invention; and
> Fig. 2 shows a generalised flow chart of the processing steps according to one embodiment of the invention.

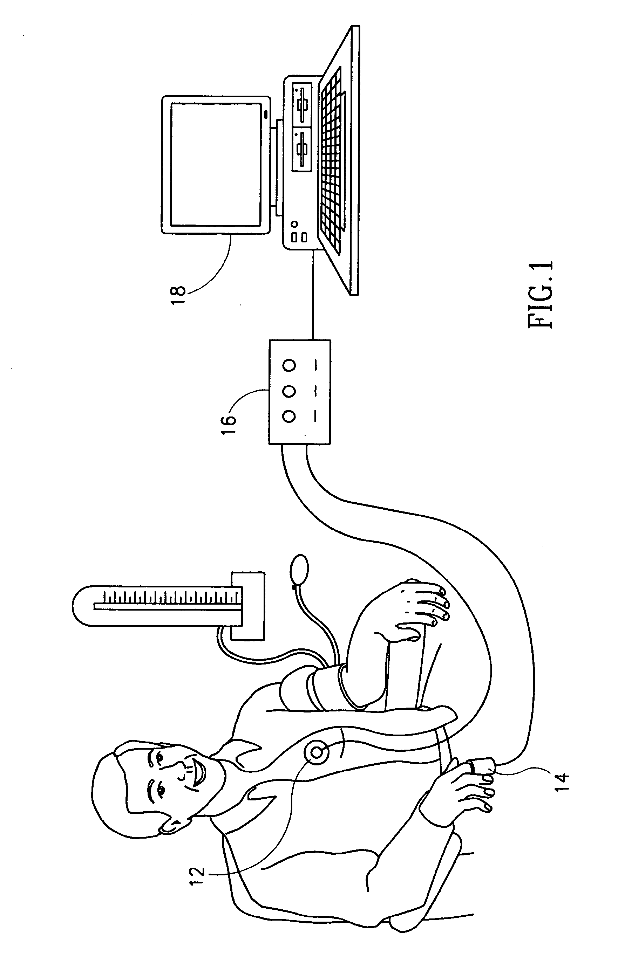

Fig. 1 shows a subject 10 being monitored by a device according to a preferred embodiment of the invention. ECG electrodes 12 have been affixed to the subject's chest for continuously and non-invasively monitoring his/her electrocardiograph. A PG sensor 14 has been attached to the subject's finger for continuously and non-invasively monitoring his/her pulse wave. Signals from the ECG electrodes and the PG sensor are continuously fed into a processor 16. The processor 16 includes an interface, an A/D converter, amplifiers and a cable to a serial port of a PC computer 18. Preliminary blood pressure measurements are carried out for calibration purposes in order to obtain any empirically defined constants using a commercially available sphygmomanometer.

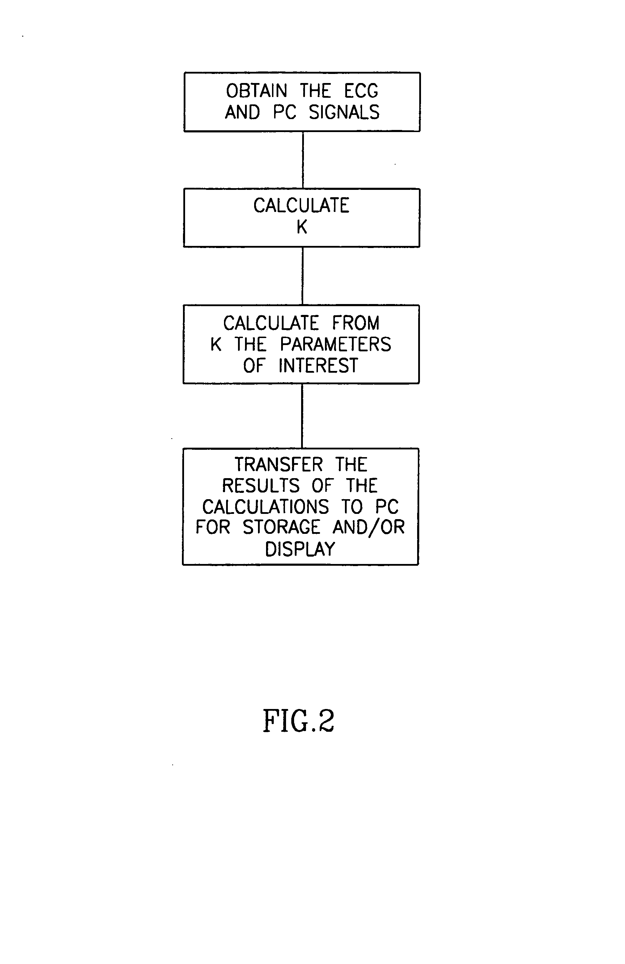

A generalised flow chart of the processing carried out by processor 16 is shown in Fig. 2. The ECG and PG signals are first processed in real time so as to obtain instantaneous values of $\kappa$. $\kappa$ is then processed in real time so as to obtain the instantaneous values of the desired parameters. The calculated values of the desired parameters are transferred in real time to PC 18 for storage and display.

Example

The invention will now be demonstrated by way of a non-limiting example.

**Methods**

The blood pressure of a group of eleven subjects, 7 males and 4 females ranging in age from 21-44, was examined using the invention. Of the 11 subjects, 10 were known to have normal blood pressure, while one had borderline hypertension. Each subject was examined at least twice. Each examination lasted approximately one hour and included measurements in the following positions: supine (15 min), sitting (15 min), and standing (10 min). In 7 subjects measurements were also made in a sitting position after 10 min of controlled physical exercise on a bicycle or during a Valsalva test. The data were processed separately for each subject and for each position.

Reference blood pressure measurements were obtained from each subject using one or both of the following devices:

> 1. A commercially available blood pressure measurement device (A Dynapulse 200M™ comprising a cuff-manometer connected to a PC computer).
> 2. Continuous oscillometric blood pressure measurement from the finger arteries (Finapress™, Ohmeda) combined with a device (Ultramind) for the transmission of the output to a PC computer.

When the Finapress™ device was used as a reference, blood pressure was measured continuously and saved in real time. When the Dynapulse™ device was used, discrete blood pressure measurements were made 3-4 times during the examination. The reference blood pressure measurements at the beginning of each examination were used to obtain the constant parameters $k_1$, $k_2$

ECG and PG signals were obtained from each subject and processed by custom software in real time. Processing included the following successive operations:

1. Smoothing (filtering and high-frequency noise).
2. Baseline drift correlation of the PG signal (high pass filtering using a cut-off frequency of 1.0-2.0 Hz).
3. Performing a peak recognition procedure on the ECG and PG signals.
4. Obtaining PA as the height of the PG peak.
5. Calculating PTT as the time interval between an ECG peak and the corresponding PG peak.
6. Calculating AREA by integration of the PG signal over the time interval from the ECG peak to the PG peak.
7. Calculating the heart rate.
8. Calculating the constant parameters $k_1$, $k_2$, $\alpha$, and $\varepsilon$ using a chi-square test in accordance with the maximal likelihood principle.

SP and DP were then obtained for each subject as follows:

ECG and PG signals were obtained from each subject and processed by custom software in real time. Processing included the following successive operations:

1. Smoothing (filtering and high-frequency noise).
2. Baseline drift correlation of the PG signal (high pass filtering using a cut-off frequency of 1.0-2.0 Hz).
3. Performing a peak recognition procedure on the ECG and PG signals.
4. Obtaining PA as the height of the PG peak.
5. Calculating PTT as the time interval between an ECG peak and the corresponding PG peak.
6. Calculating AREA by integration of the PG signal over the time interval from the ECG peak to the PG peak.
7. Calculating the heart rate.
8. Calculating SP and DP according to the methods of the invention.
9. Calculating SP and DP according to the method of European Patent Application EPO 443267A1 of Smith.

The constant parameters were adjusted from time to time during the examination as required.

The results were compared with those obtained by the following methods,

The output consisted of the following two parts:

1. SP and DP time series obtained according to the invention and according to the method of Smith.
2 The mean error and the root-mean-square error between the SP and DP time series and the reference blood pressure measurements.

## Results

The blood pressure measurements obtained according to the invention on subjects at rest, and those calculated by the empirical formulas of European Patent Application EPO 443267A1 of Smith (Table 1) were compared with those obtained by the reference devices. SP and DP determinations obtained according to the present invention are more stable than those obtained by the method of Smith. In particular, the mean error and standard deviations of SP measurements obtained after stress according to Methods 3 and 5 of the present invention were 2 and 5 times smaller, respectively, than those obtained by the method of Smith. In all 26 subjects, when SP measurements were obtained according to Method 3 and 5 the mean error was 1.6 times smaller than that obtained by the method of Smith (p=0.023).

Table 2 shows the results of blood pressure measurements obtained on subjects while supine or sitting after exercise. The five methods of the invention and the method of European patent Application EPO 443267A1 of Smith were compared with measurements obtained by Finapress™ and a Dynapulse 200M™. In particular, the mean error between SP measurements obtained according to Methods 3 and 5 in all 26 subjects was 54% of the error obtained of Smith (p=0.023).

**Table 1**

| Subject's position | | Method 1 | Methods 2, 4 | Methods 3, 5 | Smith |
|---|---|---|---|---|---|
| Dynapulse™ (n=12) | SP | 18±13 | 13±8 | 10±8 | 20±37 |
| | DP | 11±8 | 7±5 | 6±4 | 8±4 |
| Finapress™ (n=14) | SP | 12±5 | 9±4 | 8±4 | 9±4 |
| | DP | 7±3 | 6±3 | 8±4 | 6±3 |

**Table 1.** Mean error ± standard deviation (mm Hg) between the blood pressure measurements obtained by the five methods of the present invention, and the Method of Smith compared with measurements obtained using a Finapress™ or Dynapulse™.

**Table 2**

| Subject's position | | Method 1 | Methods 2, 4 | Methods 3,5 | Smith |
|---|---|---|---|---|---|
| Supine, (n=19) | SP | 11±5 | 9±4 | 7±4 | 8±4 |
| | DP | 7±4 | 6±4 | 7±4 | 6±3 |
| Sitting, following exercise (N = 7+) | SP | 23±14 | 17±8 | 14±9 | 30±47 |
| | DP | 14±8 | 7±4 | 7±4 | 8±4 |
| Total (n=26) | SP | 15±10 | 11±6 | 9±6 | 14±26 |
| | DP | 9±6 | 6±4 | 7±4 | 7±3 |

Table 2. Mean error ± standard deviation (mm Hg) obtained by the five methods of the present invention and the method of European Patent Application EP-A-443267 of Smith compared with measurements obtained using a Finapress™ and a Dynapulse 200M™. The measurements were made while the subject was either supine with no prior exercise or sitting following exercise.

**Claims**

1. A method for obtaining continuously and non-invasively one or more of the parameters of a subject from the list comprising:

   i. systolic blood pressure,
   ii. diastolic blood pressure,
   iii. Young modulus of an artery,
   iv. cardiac output,
   v. relative changes in vascular resistance, and
   vi. relative changes in vascular compliance; said method comprising:

   (a) substantially obtaining continuously and non-invasively the ratio, $\kappa$, of the subject's blood flow velocity to the propagation speed of the subject's pulse wave; and
   (b) processing $\kappa$ substantially in real time so as to obtain the instantaneous values of the desired parameters.

2. The method according to Claim 1, wherein $\kappa$ is obtained by processing a plethysmograph PG signal and a pulse transit time PTT continuously and non-invasively obtained from the subject.

3. The method of Claim 2 wherein $\kappa$ is obtained according to the following algorithmic expression:

$$\kappa = 1/(1/(PEAK \cdot v) + 1),$$

where v is the pulse velocity, and

$$PEAK = k_1 \cdot PTTPA + k_2 \cdot AREA,$$

where PA and AREA are respectively the amplitude and area of the pulse wave obtained from the PG signal and $k_1$ and $k_2$ are obtained empirically.

4. The method of Claim 2, wherein $\kappa$ is obtained according to the following algorithm expression:

$$\kappa = \frac{1}{\left(\left(\dfrac{1}{PA}\right)+1\right)}$$

where PA is the amplitude of the pulse wave obtained from the PG signal.

5. The method of Claims 3 and 4 further comprising filtering out slow fluctuations in the pulse wave.

6. The method of Claim 5 wherein slow fluctuations in PEAK are filtered out by replacing PEAK in Claim 3 with PEAK/ (slow component of PEAK)$^2$

7. The method of Claim 1, wherein the processing stipulated in step (a) for the calculation of the subject's systolic blood pressure includes the algorithmic expression

$$SP = \rho v^2 \Phi(\kappa, \gamma),$$

where $\rho$ is the blood density, $\gamma$ is the thermodynamic Poisson exponent of the blood, v is the pulse wave velocity and

$$\Phi = \frac{\sqrt{2\kappa(\gamma-1)^2 + 4\cdot(\gamma-1)+1}-1}{2(\gamma-1)}$$

8. The method of Claim 1, wherein the processing stipulated in step (a) for the calculation of the subject's systolic blood pressure includes the algorithmic expression

$$SP = (\log v^2)/\alpha + 2\rho\, v^2 \kappa/3 + \lambda,$$

where $\rho$ is the blood density, v is the pulse wave velocity, and $\lambda = (\log(2\rho R/E_0 h))/\alpha$, where R is the radius of the artery, $E_0$ is Young modulus referred to zero pressure, h is the thickness of the arterial wall and $\alpha$ is obtained empirically.

9. The method of Claim 1. wherein the processing stipulated in step (a) for the calculation of the subject's systolic blood pressure includes the algorithmic expression

$$SP = [(\log v^2)/\alpha + \lambda]/(1 - \kappa)$$

where v is the pulse wave velocity and $\lambda = (\log (2\rho R/E_0 h))/\alpha$, where R is the radius of the artery, $E_0$ is Young modulus referred to zero pressure, $\rho$ is the blood density, h is the thickness of the arterial wall and $\alpha$ is obtained empirically.

**10.** The method of Claim 1, wherein the processing stipulated in step (a) for the calculation of the subject's systolic blood pressure includes the algorithmic expression

$$SP = (\log v^2/(1 - \varepsilon H^2))/\alpha + 2\rho v^2 \kappa/3 + \lambda$$

where $\rho$ is the blood density, v is the pulse wave velocity, H is the heart rate, $\lambda = (\log (2\rho R/E_0 h))/\alpha$, where R is the radius of the artery, $E_0$ is Young modulus referred to zero pressure, h is the thickness of the arterial wall and $\varepsilon$ and $\alpha$ are obtained empirically.

**11.** The method of Claim 1, wherein the processing stipulated in step (a) for the calculation of the subject's systolic blood pressure includes the algorithmic expression

$$SP = [(\log v^2/(1 - \varepsilon H^2))/\alpha + \lambda]/(1 - \kappa),$$

where, v is the pulse wave velocity, H is the heart rate, $\lambda = (\log (2\rho R/E_0 h))/\alpha$, where R is the radius of the artery, $E_0$ is Young modulus referred to zero pressure, h is the thickness of the arterial wall, $\rho$ is the blood density, and $\varepsilon$ and $\alpha$ are obtained empirically.

**12.** The method of Claim 1, wherein the processing stipulated in step (a) for the calculation of the subject's diastolic blood pressure includes the algorithmic expression

$$DP = SP - \rho v^2 \kappa,$$

where SP is the systolic pressure, $\rho$ is the blood density, and v is the pulse wave velocity.

**13.** The method of Claim 1, wherein the processing stipulated in step (a) for the calculation of Young modulus of an artery of the subject includes the algorithmic expression

$$E = (2R/h) (SP\text{-}DP)/\kappa$$

where R is the radius of the artery, h is the thickness of the arterial wall, SP is the systolic pressure and DP is the diastolic pressure.

**14.** The method of Claim 1, wherein the processing stipulated in step (a) for the calculation of Young modulus of an artery of the subject includes the algorithmic expression

$$E = (2R/h) SP/\Phi (\kappa, \gamma)$$

where R is the radius of the artery, h is the thickness of the arterial wall, SP is the systolic pressure, $\gamma$ is the thermodynamic Poisson exponent of the blood and

$$\Phi = \frac{\sqrt{2\kappa(\gamma-1)^2 + 4\cdot(\gamma-1)+1} - 1}{2(\gamma-1)}$$

15. The method of Claim 1, wherein the processing stipulated in step (a) for the calculation of Young modulus of an artery of the subject includes the algorithmic expression

$$E = (2R/h)\cdot\rho\cdot\exp{(-\lambda + MP)\alpha}$$

where R is the radius of the artery, h is the thickness of the arterial wall, $\rho$ is the blood density, $MP = (SP + 2\cdot DP)/3$ where SP is the systolic pressure, DP is the diastolic pressure wherein at least one of the systolic pressure or the diastolic pressure is obtained using an algorithmic expression involving $\kappa$, and $\lambda = (\log{(2\rho R/E_0 h)})/\alpha$ where $E_0$ is Young modulus referred to zero pressure and $\alpha$ is an empirically obtained constant.

16. The method of Claim 1, wherein the processing stipulated in step (a) for the calculation of Young modulus of an artery of the subject includes the algorithmic expression

$$E = (2R/h)\cdot\rho\cdot\exp{((-\lambda + SP\cdot(1-\kappa))\alpha)}$$

where R is the radius of the artery, h is the thickness of the artery wall, $\rho$ is the blood density, SP is the systolic pressure and $\lambda = (\log{(2\rho R/E_o h)})/\alpha$, wherein $E_o$, is Young Modulus referred to zero pressure and $\alpha$ is an empirically obtained constant.

17. The method of Claim 1, wherein the processing stipulated in step (a) for the calculation of the relative change in the cardiac output of a subject includes the algorithmic expression

$$CO = PEAK\cdot\{v\cdot[1 + SP/(2\rho\cdot v^2)]\}^2$$

where SP is a systolic pressure obtained using an algorithmic expression involving $\kappa$, $\rho$ is the blood density, and v is the pulse wave velocity and

$$PEAK = k_1\cdot PTT\cdot PA + k_2\cdot AREA,$$

where PA and AREA are respectively the amplitude and area of the pulse wave peak obtained from a PG signal, and $k_1$ and $k_2$ are obtained empirically.

18. The method of Claim 14 further comprising filtering out slow fluctuations in the pulse wave.

19. The method of Claim 1, wherein the processing stipulated in step (a) for the calculation of the subject's cardiac resistance includes the algorithmic expression

$$VR = (SP - DP)/CO.$$

where any one or more of SP, DP, and CO are obtained from a calculation involving κ.

20. The method of Claim 1, wherein the processing stipulated in step (a) for the calculation of the relative change in the vascular compliance of a subject includes the algorithmic expression

$$VC = PEAK/(SP - DP),$$

Where

$$PEAK = k_1 \cdot PTT \cdot PA + k_2 \cdot AREA,$$

where PA and AREA are respectively the amplitude and area of the pulse wave obtained from the PG signal, and $k_1$ and $k_2$ are obtained empirically.

21. A method for determining continuously and non-invasively whether a change in a subject's blood pressure is due to a change in cardiac output or vascular compliance, comprising:

(a) substantially obtaining continuously and non-invasively the ratio, κ of the subject's blood flow velocity to the propagation speed of the subject's pulse wave;
(b) processing κ substantially in real time so as to obtain the instantaneous values of the subject's SP, CO and VC; and
(c) processing the subject's SP, CO, and VC in real time so as to obtain the instantaneous values of the algorithmic expression:

$$INDEX1 = \partial SP/\partial CO - \partial SP/\partial VC,$$

an increase in INDEX1 over time indicating a change in the subject's blood pressure due to a change in cardiac output, otherwise the change in the subject's blood pressure is due to a change in vascular compliance.

22. A method for determining continuously and non-invasively whether a change in a subject's blood pressure is due to a change in the subject's cardiac output or vascular resistance, comprising:

(a) substantially obtaining continuously and non-invasively the ratio, κ of the subject's blood flow velocity to the propagation speed of the subject's pulse wave;
(b) processing κ substantially in real time so as to obtain the instantaneous values of the subject's SP, CO and VR; and
(c) processing the subject's SP, CO, and VR in real time so as to obtain the instantaneous values of the algorithmic expression:

$$INDEX2 = \partial SP/\partial CO - \partial SP/\partial VR$$

an increase in INDEX2 over time indicating a change in the subject's blood pressure due to a change in cardiac output, otherwise the change in the subject's blood pressure is due to a change in vascular resistance.

23. A device for obtaining continuously and non-invasively one or more of the vascular parameters of a subject (10) from the list comprising:

i. systolic blood pressure,
ii. diastolic blood pressure,
iii. Young's modulus of an artery,

iv. relative change in cardiac output,
v. relative change in vascular resistance, and
vi. relative changes in vascular compliance;

said device comprising:

(a) a device substantially obtaining continuously and non-invasively the ratio $\kappa$ of the subject's blood flow velocity to the propagation speed of the subject's pulse wave; and
(b) a device (16) processing $\kappa$ substantially in real time so as to obtain the instantaneous values of the desired parameters.

24. The device according to Claim 23, wherein $\kappa$ is obtained by processing a plethysmograph PG and a pulse transit time PTT continuously and non-invasively obtained from the subject (10).

25. The device of Claim 24 wherein $\kappa$ is obtained according to the following algorithmic expression:

$$\kappa = 1/(1/(PEAK \cdot v) + 1),$$

where v is inversely proportional to PTT, and

$$PEAK = k_1 \cdot PTT \cdot PA + k_2 \cdot AREA,$$

where PA and AREA are respectively the amplitude and area of the pulse wave obtained from the PG signal and $k_1$ and $k_2$ are obtained empirically.

26. The device according to Claim 23, wherein $\kappa$ is obtained according to the following algorithmic expression

$$\kappa = 1/((1/PA) + 1)$$

where PA is the amplitude of the pulse wave obtained from the PG signal.

27. The device of Claim 25 capable of filtering out slow fluctuations in the pulse wave.

28. The device of Claim 27 wherein slow fluctuations in the pulse wave are filtered out by replacing PEAK in Claim 22 with PEAK/(slow component of PEAK)$^2$

29. The device of Claim 23, wherein the processing stipulated in step (a) for the calculation of the subject's systolic blood pressure includes the algorithmic expression

$$SP = \rho v^2 \Phi(\kappa, \gamma),$$

where $\rho$ is the blood density, $\gamma$ is the thermodynamic Poisson exponent of the blood, v is the pulse wave velocity and

$$\Phi = \frac{\sqrt{2\kappa(\gamma-1)^2 + 4 \cdot (\gamma-1) + 1} - 1}{2(\gamma-1)}$$

30. The device of Claim 23, wherein the processing stipulated in step (a) for the calculation of the subject's systolic blood pressure includes the algorithmic expression

$$SP = (\log v^2)/\alpha + 2\rho\, v^2\kappa/3 + \lambda,$$

where $\rho$ is the blood density, and $\lambda = (\log 2\rho R/E_0 h)/\alpha$, where R is the radius of the artery, $E_0$ is Young modulus referred to zero pressure, h is the thickness of the arterial wall and $\alpha$ is obtained empirically.

**31.** The device of Claim 23, wherein the processing stipulated in step (a) for the calculation of the subject's systolic blood pressure includes the algorithmic expression

$$SP = [(\log v^2)/\alpha + \lambda]/(1 - \kappa)$$

where v is the pulse wave velocity and $\lambda = (\log (2\rho R/E_0 h))/\alpha$, where R is the radius of the artery, $E_0$ is Young modulus referred to zero pressure, $\rho$ is the blood density, h is the thickness of the arterial wall and $\alpha$ is obtained empirically.

**32.** The device of Claim 23, wherein the processing stipulated in step (a) for the calculation of the subject's systolic blood pressure includes the algorithmic expression

$$SP = (\log v^2/(1 - \varepsilon H^2))/\alpha + 2\rho v^2\kappa/3 + \lambda$$

where $\rho$ is the blood density, v is the pulse wave velocity, H is the heart rate, $\lambda = (\log (2\rho R/E_0 h))/\alpha$, where R is the radius of the artery, $E_0$ is Young modulus referred to zero pressure, h is the thickness of the arterial wall and $\varepsilon$ and $\alpha$ are obtained empirically.

**33.** The device of Claim 23, wherein the processing stipulated in step (a) for the calculation of the subject's systolic blood pressure includes the algorithmic expression

$$SP = [(\log v^2/(1 - \varepsilon H^2))/\alpha + \lambda]/(1 - \kappa),$$

where, v is the pulse wave velocity, H is the heart rate, $\lambda = (\log (2\rho R/E_0 h))/\alpha$, where R is the radius of the artery, $E_0$ is Young modulus referred to zero pressure, h is the thickness of the arterial wall, $\rho$ is the blood density, and $\varepsilon$ and $\alpha$ are obtained empirically.

**34.** The device of Claim 23, wherein the processing stipulated in step (a) for the calculation of the subject's diastolic blood pressure includes the algorithmic expression

$$DP = SP - \rho v^2\kappa,$$

where SP is the systolic pressure $\rho$ is the blood density, and v is the pulse wave velocity.

**35.** The device of Claim 23, wherein the processing stipulated in step (a) for the calculation of Young modulus of an artery of the subject includes the algorithmic expression

$$E = (2R/h)\, (SP\text{-}DP)/\kappa$$

where R is the radius of the artery, h is the thickness of the arterial wall, SP is the systolic pressure and DP is the diastolic pressure.

36. The device of Claim 23, wherein the processing stipulated in step (a) for the calculation of Young modulus of an artery of the subject includes the algorithmic expression

$$E = (2R/h)\ SP/\Phi\ (\kappa,\gamma)$$

where R is the radius of the artery, h is the thickness of the arterial wall, SP is the systolic pressure, $\gamma$ is the thermodynamic Poisson exponent of the blood and

$$\Phi = \frac{\sqrt{2\kappa(\gamma-1)^2 + 4\cdot(\gamma-1)+1} - 1}{2(\gamma-1)}$$

37. The device of Claim 23, wherein the processing stipulated in step (a) for the calculation of Young modulus of an artery of the subject includes the algorithmic expression

$$E = (2R/h)\cdot\rho\cdot\exp\ (-\lambda+MP)\alpha$$

where R is the radius of the artery, h is the thickness of the arterial wall, $\rho$ is the blood density, MP = (SP+ 2-DP)/ 3 where SP is the systolic pressure, DP is the diastolic pressure wherein at least one of the systolic pressure or the diastolic pressure is obtained using an algorithmic expression involving $\kappa$, and $\lambda$ = (log $(2\rho R/E_o h))/\alpha$ where $E_o$ is Young modulus referred to zero pressure and $\alpha$ is an empirically obtained constant.

38. The device of Claim 35, wherein the processing stipulated in step (a) for the calculation of Young modulus of an artery of the subject includes the algorithmic expression

$$E = (2R/h)\cdot\rho\cdot\exp\ ((-\lambda+SP\cdot(1-\kappa))\alpha$$

where R is the radius of the artery, h is the thickness of the artery wall, $\rho$ is the blood density, SP is the systolic pressure and $\lambda$ = (log $(2\rho R/E_o h))/\alpha$, wherein $E_o$ is Young Modulus referred to zero pressure and $\alpha$ is an empirically obtained constant.

39. The device of Claim 23, wherein the processing stipulated in step (a) for the calculation of the relative change in the cardiac output of a subject includes the algorithmic expression

$$CO = PEAK \cdot \{v \cdot [1 + SP/(2\rho\cdot v^2)]\}^2$$

where SP is a systolic pressure obtained using an algorithmic expression involving $\kappa$ and

$$PEAK = k_1\cdot PTT\cdot PA + k_2\cdot AREA,$$

where PA and AREA are respectively the amplitude and area of the pulse wave obtained from a PG signal, and $k_1$ and $k_2$ are obtained empirically.

40. The device of Claim 23 wherein the processing stipulated in step (a) for the calculation of the subject's cardiac resistance includes the algorithmic expression

$$VR = (SP - DP)/CO.$$

where any one or more of SP, DP and CO are obtained from a calculation involving $\kappa$.

41. A device determining continuously and non-invasively whether a change in a subject's blood pressure is due to a change in cardiac output or vascular compliance, comprising:

(a) a device substantially obtaining continuously and non-invasively the ratio $\kappa$ of the subject's blood flow velocity to the propagation speed of the subject's pulse wave;
(b) a device (16) processing $\kappa$ substantially in real time so as to obtain the instantaneous values of the subject's SP, CO and VC; and
(c) a device processing the subject's SP, CO and VC in real time so as to obtain the instantaneous values of the algorithmic expression:

$$INDEX1 = \partial SP/\partial CO - \partial SP/\partial VC,$$

an increase in INDEX1 over time indicating a change in the subject's blood pressure due to a change in cardiac output, otherwise the change in the subject's blood pressure is due to a change in vascular compliance.

42. A device determining continuously and non-invasively whether a change in a subject's blood pressure is due to a change in the subject's vascular resistance comprising:

(a) a device substantially obtaining continuously and non-invasively the ratio $\kappa$ of the subject's blood flow velocity to the propagation speed of the subject's pulse wave;
(b) a device (16) processing $\kappa$ substantially in real time so as to obtain the instantaneous values of the subject's SP, CO and VR; and
(c) a device processing the subject's SP, CO and VR in real time so as to obtain the instantaneous values of the algorithmic expression:

$$INDEX2 = \partial SP/\partial CO - \partial SP/\partial VR,$$

an increase in INDEX2 over time indicating a change in the subject's blood pressure due to a change in cardiac output, otherwise the change in the subject's blood pressure is due to a change in vascular resistance.

**Patentansprüche**

1. Verfahren zum kontinuierlichen und nicht-invasiven Feststellen eines oder mehrerer Parameter einer Person aus der nachfolgenden Liste

i. systolischer Blutdruck,

ii. diastolischer Blutdruck,
iii. Elastizitätsmodul einer Arterie,
iv. Herzminutenvolumen,
v. relative Änderungen im Gefäßwiderstand und
vi. relative Änderungen in der Gefäßdehnbarkeit,

wobei das Verfahren die Stufen umfaßt:

(a) Im Wesentlichen kontinuierliches und nichtinvasives Feststellen des Verhältnisses $\kappa$ der Blutströmungsgeschwindigkeit der Person zur Ausbreitungsgeschwindigkeit der Pulswelle der Person;
(b) Verarbeiten von $\kappa$ im Wesentlichen in Echtzeit, um die Momentanwerte der gewünschten Parameter zu erhalten.

2. Verfahren nach Anspruch 1, worin $\kappa$ durch Verarbeiten eines von der Person kontinuierlich und nicht-invasiv erhaltenen Plethysmographsignals (eines PG-Signals) und einer Pulsübergangszeit (PTT) festgestellt wird.

3. Verfahren nach Anspruch 2., worin $\kappa$ gemäß dem folgenden algorithmischen Ausdruck

$$\kappa = 1 / (1 / (PEAK \cdot v) + 1)$$

erhalten wird, worin v die Pulsgeschwindigkeit und

$$PEAK = k_1 \cdot PTT \cdot PA + k_2 \cdot FLÄCHE$$

ist, wobei PA und FLÄCHE die Amplitude bzw. die Fläche der Pulswelle, die aus dem PG-Signal erhalten wird, bedeuten sowie $k_1$ und $k_2$ sich empirisch ergeben.

4. Verfahren nach Anspruch 2, worin $\kappa$ gemäß dem folgenden algorithmischen Ausdruck

$$\kappa = \frac{1}{\left(\left(\frac{1}{PA}\right) + 1\right)}$$

erhalten wird, worin PA die Amplitude der aus dem PG-Signal sich ergebenden Pulswelle bedeutet.

5. Verfahren nach Anspruch 3 und 4, das ferner das Ausfiltern von langsamen Fluktuationen in der Pulswelle beinhaltet.

6. Verfahren nach Anspruch 5, worin langsame Fluktuationen im PEAK durch Ersetzen von PEAK im Anspruch 3 durch PEAK/(langsame Komponente von PEAK)$^2$ ausgefiltert werden.

7. Verfahren nach Anspruch 1, worin das in der Stufe (a) festgelegte Verarbeiten für die Berechnung des systolischen Blutdrucks der Person den algorithmischen Ausdruck

$$SP = \rho v^2 \Phi(\kappa, \gamma)$$

beinhaltet, worin $\rho$ die Blutdichte, $\gamma$ der thermodynamische Poisson-Exponent des Bluts, v die Pulswellengeschwindigkeit und

$$\Phi = \frac{\sqrt{2\kappa(\gamma - 1)^2 + 4 \cdot (\gamma - 1) + 1} - 1}{2(\gamma - 1)}$$

ist.

8.  Verfahren nach Anspruch 1, worin das in der Stufe (a) festgelegte Verarbeiten für die Berechnung des systolischen Blutdrucks der Person den algorithmischen Ausdruck

$$SP = (\log v^2)/\alpha + 2\rho\, v^2\kappa/3 + \lambda$$

beinhaltet, worin $\rho$ die Blutdichte, v die Pulswellengeschwindigkeit und $\lambda = (\log(2\rho R/E_0 h))/\alpha$, ist, wobei R den Radius der Arterie, $E_0$ den auf den Nulldruck bezogenen Elastizitätsmodul und h die Dicke der Arterienwand bedeuten sowie $\alpha$ empirisch erhalten wird.

9.  Verfahren nach Anspruch 1 , worin das in der Stufe (a) festgelegte Verarbeiten für die Berechnung des systolischen Blutdrucks der Person den algorithmischen Ausdruck

$$SP = [(\log v^2)/\alpha + \lambda]/(1 - \kappa)$$

beinhaltet, worin v die Pulswellengeschwindigkeit und $\lambda = (\log(2\rho R/E_0 h))/\alpha$ ist, wobei R den Radius der Arterie, $E_0$ den auf den Nulldruck bezogenen Elastizitätsmodul, $\rho$ die Blutdichte und h die Dicke der Arterienwand bedeuten sowie $\alpha$ empirisch erhalten wird.

10.  Verfahren nach Anspruch 1, worin das in der Stufe (a) festgelegte Verarbeiten für die Berechnung des systolischen Blutdrucks der Person den algorithmischen Ausdruck

$$SP = (\log v^2/(1 - \varepsilon H^2))/\alpha + 2\rho v^2\kappa/3 + \lambda$$

beinhaltet, worin $\rho$ die Blutdichte, v die Pulswellengeschwindigkeit, H die Herzfrequenz und $\lambda = (\log(2\rho R/E_0 h))/\alpha$ ist, wobei R den Radius der Arterie, $E_0$ den auf den Nulldruck bezogenen Elastizitätsmodul und h die Dicke der Arterienwand bedeuten sowie $\varepsilon$ und $\alpha$ empirisch erhalten werden.

11.  Verfahren nach Anspruch 1, worin das in der Stufe (a) festgelegte Verarbeiten für die Berechnung des systolischen Blutdrucks der Person den algorithmischen Ausdruck

$$SP = [(\log v^2/(1 - \varepsilon H^2))/\alpha + \lambda]/(1 - \kappa)$$

beinhaltet, worin v die Pulswellengeschwindigkeit, H die Herzfrequenz und $\lambda = (\log(2\rho R/E_0 h))/\alpha$ ist, wobei R den Radius der Arterie, $E_0$ den auf den Nulldruck bezogenen Elastizitätsmodul, h die Dicke der Arterienwand und $\rho$ die Blutdichte bedeuten sowie $\varepsilon$ und $\alpha$ empirisch erhalten werden.

12. Verfahren nach Anspruch 1, worin das in der Stufe (a) festgelegte Verarbeiten für die Berechnung des diastolischen Blutdrucks der Person den algorithmischen Ausdruck

$$DP = SP - \rho v^2 \kappa$$

beinhaltet, worin SP der systolische Druck, $\rho$ die Blutdichte und v die Pulswellengeschwindigkeit bedeuten.

13. Verfahren nach Anspruch 1, worin das in der Stufe (a) festgelegte Verarbeiten für die Berechnung des Elastizitätsmoduls einer Arterie der Person den algorithmischen Ausdruck

$$E = (2R/h)(SP-DP)/\kappa$$

beinhaltet, worin R den Radius der Arterie, h die Dicke der Arterienwand, SP den systolischen Druck und DP den diastolischen Druck bedeuten.

14. Verfahren nach Anspruch 1, worin das in der Stufe (a) festgelegte Verarbeiten für die Berechnung des Elastizitätsmoduls einer Arterie der Person den algorithmischen Ausdruck

$$E = (2R/h)SP/\Phi(\kappa,\gamma)$$

beinhaltet, worin R der Radius der Arterie, h die Dicke der Arterienwand, SP der systolische Druck, $\gamma$ der thermodynamische Poisson-Exponent des Bluts und

$$\Phi = \frac{\sqrt{2\kappa(\gamma - 1)^2 + 4 \cdot (\gamma - 1) + 1} - 1}{2(\gamma-1)}$$

ist.

15. Verfahren nach Anspruch 1, worin das in der Stufe (a) festgelegte Verarbeiten für die Berechnung des Elastizitätsmoduls einer Arterie der Person den algorithmischen Ausdruck

$$E = (2R/h) \cdot \rho \cdot \exp(-\lambda + MP)\alpha$$

beinhaltet, worin R der Radius der Arterie, h die Dicke der Arterienwand, $\rho$ die Blutdichte und MP = (SP+ 2.DP)/3 ist, wobei SP den systolischen Druck und DP den diastolischen Druck bedeuten und mindestens der systolische Druck oder der diastolische Druck unter Anwendung eines algorithmischen Ausdrucks erhalten worden ist, der $\kappa$ enthält, sowie $\lambda = (\log(2\rho R/E_0 h))/\alpha$ ist, worin $E_0$ den auf den Nulldruck bezogenen Elastizitätsmodul bedeutet und $\alpha$ eine empirische erhaltene Konstante ist.

16. Verfahren nach Anspruch 1, worin das in der Stufe (a) festgelegte Verarbeiten für die Berechnung des Elastizitätsmoduls einer Arterie der Person den algorithmischen Ausdruck

$$E = (2R/h) \cdot \rho \cdot \exp((-\lambda + SP \cdot (1-\kappa))\alpha)$$

beinhaltet, worin R der Radius der Arterie, h die Dicke der Arterienwand, $\rho$ die Blutdichte, SP der systolische Druck und $\lambda = (\log(2\rho R/E_0 h))/\alpha$ ist, wobei $E_0$ den auf den Nulldruck bezogenen Elastizitätsmodul darstellt und $\alpha$ eine empirisch erhaltene Konstante bedeutet.

17. Verfahren nach Anspruch 1, worin das in der Stufe (a) festgelegte Verarbeiten für die Berechnung der relativen Änderung des Herzminutenvolumens einer Person den algorithmischen Ausdruck

$$CO = PEAK \cdot \{v \cdot [1+SP/(2\rho\ v^2)]\}$$

beinhaltet, worin SP einen systolischen Druck bedeutet, der unter Anwendung eines algorithmischen Ausdrucks erhalten worden ist, der K enthält, sowie $\rho$ die Blutdichte und v die Pulswellengeschwindigkeit bedeuten sowie

$$PEAK = k_1 \cdot PTT \cdot PA + k_2 \cdot FLÄCHE$$

ist, wobei PA und FLÄCHE die Amplitude bzw. die Fläche des Pulswellenpeaks darstellen, die aus einem PG-Signal erhalten worden ist, sowie $k_1$ und $k_2$ empirisch erhalten werden.

18. Verfahren nach Anspruch 14, das ferner das Ausfiltern von langsamen Fluktuationen in der Pulswelle beinhaltet.

19. Verfahren nach Anspruch 1, worin das in der Stufe (a) festgelegte Verarbeiten für die Berechnung des Herzwiderstands der Person den algorithmischen Ausdruck

$$VR = (SP - DP)/CO$$

beinhaltet, worin eine oder mehrere der Größen SP, DP und CO aus einer Berechnung erhalten worden sind, die $\kappa$ beinhaltet.

**20.** Verfahren nach Anspruch 1, worin das in der Stufe (a) festgelegte Verarbeiten für die Berechnung der relativen Änderung der Gefäßdehnbarkeit einer Person den algorithmischen Ausdruck

$$\mathrm{VC\ =\ PEAK/(SP\ -\ DP)}$$

beinhaltet, worin

$$\mathrm{PEAK\ =\ k_1 \cdot PTT \cdot PA\ +\ k_2 \cdot FL\ddot{A}CHE}$$

ist, wobei PA und FLÄCHE jeweils die Amplitude bzw. die Fläche der Pulswelle bedeuten, die aus dem PG-Signal erhalten worden sind, sowie $k_1$ und $k_2$ sich empirisch ergeben.

**21.** Verfahren zum kontinuierlichen und nicht-invasiven Bestimmen, ob eine Änderung im Blutdruck einer Person auf einer Änderung im Herzminutenvolumen oder in der Gefäßdenbarkeit zurückzuführen ist, mit folgenden Stufen:

(a) Im Wesentlichen kontinuierliches und nichtinvasives Bestimmen des verhältnisses $\kappa$ der Blutströmungsge-schwindigkeit der Person zur Ausbreitungsgeschwindigkeit der Pulswelle der Person,
(b) Verarbeiten von $\kappa$ im Wesentlichen in Echtzeit, um die Momentanwerte der Größen SP, CO und VC der Person zu erhalten, und
(c) Verarbeiten der Größen SP, CO und VC der Person in Echtzeit, um die Momentanwerte des algorithmischen Ausdrucks

$$\mathrm{INDEX1\ =\ \partial SP/\partial CO\ -\ \partial SP/\partial VC}$$

zu erhalten, wobei eine Zunahme von INDEX1 über die Zeit eine Änderung im Blutdruck der Person aufgrund einer Änderung des Herzminutenvolumens anzeigt oder sonst die Änderung im Blutdruck der Person auf eine Änderung in der Gefäßdehnbarkeit zurückzuführen ist.

**22.** Verfahren zum kontinuierlichen und nicht-invasiven Bestimmen, ob eine Änderung im Blutdruck einer Person auf eine Änderung im Herzminutenvolumen oder im Gefäßwiderstand zurückzuführen ist mit folgenden Stufen:

(a) Im Wesentlichen kontinuierliches und nichtinvasives Bestimmen des Verhältnisses $\kappa$ der Blutströmungsge-schwindigkeit der Person zur Ausbreitungsgeschwindigkeit der Pulswelle der Person,
(b) Verarbeiten von $\kappa$ im Wesentlichen in Echtzeit, um die Momentanwerte der Größen SP, CO und VR der Person zu erhalten, und
(c) Verarbeiten der Größen SP, CO und VR der Person in Echtzeit, um die Momentanwerte des algorithmischen Ausdrucks

$$\mathrm{INDEX2\ =\ \partial SP/\partial CO\ -\ \partial SP/\partial VR}$$

zu erhalten, wobei eine Zunahme von INDEX2 über die Zeit eine Änderung im Blutdruck der Person aufgrund einer Änderung des Herzminutenvolumens anzeigt oder sonst eine Änderung im Blutdruck der Person auf eine Änderung im Gefäßwiderstand zurückzuführen ist.

**23.** Vorrichtung zum kontinuierlichen und nicht-invasiven Feststellen eines oder mehrerer Gefäßparameter einer Person (10) aus der nachfolgenden Liste

> i. systolischer Blutdruck,
> ii. diastolischer Blutdruck,
> iii. Elastizitätsmodul einer Arterie,
> iv. relative Änderung des Herzminutenvolumens,
> v. relative Änderung des Gefäßwiderstands und
> vi. relative Änderungen der Gefäßdehnbarkeit,

wobei die Vorrichtung

> (a) eine Vorrichtung aufweist, die im Wesentlichen kontinuierlich und nicht-invasiv das Verhältnis $\kappa$ der Blutströmungsgeschwindigkeit der Person zur Ausbreitungsgeschwindigkeit der Pulswelle der Person feststellt, und
> (b) eine Vorrichtung (16) aufweist, die $\kappa$ im Wesentlichen in Echtzeit verarbeitet, so daß die Momentanwerte der gewünschten Parameter erhalten werden.

**24.** Vorrichtung nach Anspruch 23, worin $\kappa$ durch Benutzen eines Plethysmographs PG und einer Pulsübergangszeit PTT, die kontinuierlich und nicht-invasiv von der Person (10) erhalten wurde, bestimmt wird.

**25.** Vorrichtung nach Anspruch 24, worin $\kappa$ gemäß dem folgenden algorithmischen Ausdruck

$$\kappa = 1/(1/(PEAK \cdot v) + 1)$$

erhalten wird, worin v umgekehrt proportional zu PTT ist, und

$$PEAK = k_1 \cdot PTT \cdot PA + k_2 \cdot FL\ddot{A}CHE$$

ist, wobei PA und FLÄCHE die Amplitude bzw. die Fläche der Pulswelle, die aus dem PG-Signal erhalten wird, bedeuten sowie $k_1$ und $k_2$ empirisch ermittelt werden.

**26.** Vorrichtung nach Anspruch 23, worin $\kappa$ gemäß dem folgenden algorithmischen Ausdruck

$$\kappa = 1/((1/(PA) + 1)$$

erhalten wird, worin PA die Amplitude der aus dem PG-Signal erhaltenen Pulswelle bedeutet.

**27.** Vorrichtung nach Anspruch 25, die in der Lage ist, langsame Fluktuationen in der Pulswelle auszufiltern.

**28.** Vorrichtung nach Anspruch 27, worin langsame Fluktuationen in der Pulswelle durch Ersetzen von PEAK im Anspruch 22 durch PEAK/(langsame Komponente von PEAK)$^2$ ausgefiltert werden.

**29.** Vorrichtung nach Anspruch 23, worin das in der Stufe (a) festgelegte Verarbeiten für die Berechnung des systolischen Blutdrucks der Person den algorithmischen Ausdruck

$$SP = \rho v^2 \Phi(\kappa, \gamma)$$

beinhaltet, worin $\rho$ die Blutdichte, $\gamma$ der thermodynamische Poisson-Exponent des Bluts, $v$ die Pulswellengeschwindigkeit und

$$\Phi = \frac{\sqrt{2\kappa(\gamma - 1)^2 + 4 \cdot (\gamma - 1) + 1} - 1}{2(\gamma - 1)}$$

ist.

30. Vorrichtung nach Anspruch 23, worin das in der Stufe (a) festgelegte Verarbeiten für die Berechnung des systolischen Blutdrucks der Person den algorithmischen Ausdruck

$$SP = (\log v^2)/\alpha + 2\rho\ v^2\kappa/3 + \lambda$$

beinhaltet, worin $\rho$ die Blutdichte und $\lambda = (\log 2\rho R/E_0 h)/\alpha$ ist, wobei R den Radius der Arterie, $E_0$ den auf den Nulldruck bezogenen Elastizitätsmodul und h die Dicke der Arterienwand bedeuten sowie $\alpha$ empirisch erhalten wird.

31. Vorrichtung nach Anspruch 23, worin das in der Stufe (a) festgelegte Verarbeiten für die Berechnung des systolischen Blutdrucks der Person den algorithmischen Ausdruck

$$SP = [(\log v^2)/\alpha + \lambda]/(1-\kappa)$$

beinhaltet, worin $v$ die Pulswellengeschwindigkeit und $\lambda = (\log(2\rho R/E_0 h))/\alpha$ ist, wobei R den Radius der Arterie, $E_0$ den auf den Nulldruck bezogenen Elastizitätsmodul, $\rho$ die Blutdichte und h die Dicke der Arterienwand bedeuten sowie $\alpha$ empirisch erhalten wird.

32. Vorrichtung nach Anspruch 23, worin das in der Stufe (a) festgelegte Verarbeiten für die Berechnung des systolischen Blutdrucks der Person den algorithmischen Ausdruck

$$SP = (\log v^2)/(1 - \varepsilon H^2))/\alpha + 2\rho v^2\kappa/3 + \lambda$$

beinhaltet, worin $\rho$ die Blutdichte, $v$ die Pulswellengeschwindigkeit, H die Herzfrequenz und $\lambda = (\log(2\rho R/E_0 h))/\alpha$ ist, wobei R den Radius der Arterie, $E_0$ den auf den Nulldruck bezogenen Elastizitätsmodul und h die Dicke der Arterienwand bedeuten sowie $\varepsilon$ und $\alpha$ empirisch erhalten werden.

33. Vorrichtung nach Anspruch 23, worin das in der Stufe (a) festgelegte Verarbeiten für die Berechnung des systolischen Blutdrucks der Person den algorithmischen Ausdruck

$$SP = [(\log v^2/(1 - \varepsilon H^2))/\alpha + \lambda]/(1 - \kappa)$$

beinhaltet, worin v die Pulswellengeschwindigkeit, H die Herzfrequenz und $\lambda = (\log(2\rho R/E_0 h))/\alpha$ ist, wobei R den Radius der Arterie, $E_0$ den auf den Nulldruck bezogenen Elastizitätsmodul, h die Dicke der Arterienwand und $\rho$ die Blutdichte bedeuten sowie $\varepsilon$ und $\alpha$ empirisch erhalten werden.

**34.** Vorrichtung nach Anspruch 23, worin das in der Stufe (a) festgelegte Verarbeiten für die Berechnung des diastolischen Blutdrucks der Person den algorithmischen Ausdruck

$$DP = SP - \rho v^2 k$$

beinhaltet, worin SP den systolischen Druck, $\rho$ die Blutdichte und v die Pulswellengeschwindigkeit bedeuten.

**35.** Vorrichtung nach Anspruch 23, worin das in der Stufe (a) festgelegte Verarbeiten für die Berechnung des Elastizitätsmoduls einer Arterie der Person den algorithmischen Ausdruck

$$E = (2R/h)(SP - DP)/\kappa$$

beinhaltet, worin R den Radius der Arterie, h die Dicke der Arterienwand, SP den systolischen Druck und DP den diastolischen Druck bedeuten.

**36.** Vorrichtung nach Anspruch 23, worin das in der Stufe (a) festgelegte Verarbeiten für die Berechnung des Elastizitätsmoduls einer Arterie der Person den algorithmischen Ausdruck

$$E = (2R/h)SP/\Phi(\kappa, \gamma)$$

beinhaltet, worin R der Radius der Arterie, h die Dicke der Arterienwand, SP der systolische Druck, $\gamma$ der thermodynamische Poisson-Exponent des Bluts und

$$\Phi = \frac{\sqrt{2\kappa(\gamma - 1)^2 + 4 \cdot (\gamma - 1) + 1} - 1}{2(\gamma-1)}$$

ist.

**37.** Vorrichtung nach Anspruch 23, worin das in der Stufe (a) festgelegte Verarbeiten für die Berechnung des Elastizitätsmodus einer Arterie der Person den algorithmischen Ausdruck

$$E \ = \ (2R/h) \cdot \rho \cdot \exp \ (-\lambda + MP)\alpha$$

beinhaltet, worin R der Radius der Arterie, h die Dicke der Arterienwand, $\rho$ die Blutdichte und MP = (SP+ 2. DP) /3 ist, wobei SP den systolischen Druck und DP den diastolischen Druck bedeuten und mindestens der systolische Druck oder der diastolische Druck durch Benutzen eines algorithmischen Ausdrucks, der $\kappa$ enthält, erhalten wird, sowie $\lambda = (\log(2\rho R/E_0 h))/\alpha$ ist, worin $E_0$ den auf den Nulldruck bezogenen Elastizitätsmodul bedeutet und $\alpha$ eine empirisch erhaltene Konstante ist.

**38.** Vorrichtung nach Anspruch 35, worin das in der Stufe (a) festgelegte Verarbeiten für die Berechnung des Elastizitätsmoduls einer Arterie der Person den algorithmischen Ausdruck

$$E \ = \ (2R/h) \cdot \rho \cdot \exp \ ((-\lambda + SP \cdot (1 - \kappa))\alpha$$

beinhaltet, worin R der Radius der Arterie, h die Dicke der Arterienwand, $\rho$ die Blutdichte, SP der systolische Druck und $\lambda = (\log (2\rho R/E_0 h))/\alpha$ ist, wobei $E_0$ den auf den Nulldruck bezogenen Elastizitätsmodul bedeutet und $\alpha$ eine empirisch erhaltene Konstante ist.

**39.** Vorrichtung nach Anspruch 23, worin das in der Stufe (a) festgelegte Verarbeiten für die Berechnung der relativen Änderung des Herzminutenvolumens einer Person den algorithmischen Ausdruck

$$CO \ = \ PEAK \cdot \left\{ v \cdot [1 \ + \ SP/(2\rho \cdot v^2)] \right\}^2$$

beinhaltet, worin SP der systolische Druck, welcher unter Benutzung eines algorithmischen Ausdrucks, der $\kappa$ enthält, erhalten wurde, und

$$PEAK \ = \ k_1 \cdot PTT \cdot PA \ + \ k_2 \cdot FL\ddot{A}CHE$$

ist, wobei PA und FLÄCHE die Amplitude bzw. die Fläche der von einem PG-Signal erhaltenen Pulswelle bedeuten sowie $k_1$ und $k_2$ empirisch erhalten werden.

**40.** Vorrichtung nach Anspruch 23, worin das in der Stufe (a) festgelegte Verarbeiten für die Berechnung des Herzwiderstands der Person den algorithmischen Ausdruck

$$VR \ = \ (SP \ - \ DP)/CO$$

beinhaltet, worin eine oder mehrere der Größen SP, DP und CO aus einer Berechnung erhalten worden ist, die $\kappa$ beinhaltet.

**41.** Vorrichtung zum kontinuierlichen und nicht-invasiven Bestimmen, ob eine Änderung im Blutdruck einer Person auf eine Änderung im Herzminutenvolumen oder in der Gefäßdehnbarkeit zurückzuführen ist, wobei die Vorrichtung

(a) eine Vorrichtung aufweist, die im Wesentlichen kontinuierlich und nicht-invasiv das Verhältnis κ der Blutströmungsgeschwindigkeit der Person zur Ausbreitungsgeschwindigkeit der Pulswelle der Person feststellt,
(b) eine Vorrichtung (16) aufweist, die κ im Wesentlichen in Echtzeit verarbeitet, so daß die Momentanwerte der Größen SP, CO und VC der Person erhalten werden, und
(c) eine Vorrichtung aufweist, welche die Größen SP, CO und VC der Person in Echtzeit verarbeitet, so daß die Momentanwerte des algorithmischen Ausdrucks

$$\mathtt{INDEX1} = \partial SP/\partial CO - \partial SP/\partial VC$$

erhalten werden, wobei eine Zunahme von INDEX1 über die Zeit auf eine Änderung des Blutdrucks der Person aufgrund einer Änderung des Herzminutenvolumens anzeigt oder sonst die Änderung im Blutdruck der Person auf einer Änderung der Gefäßdehnbarkeit beruht.

42. Vorrichtung zum kontinuierlichen und nicht-invasiven Bestimmen, ob eine Änderung im Blutdruck einer Person auf eine Änderung des Gefäßwiderstands der Person zurückzuführen ist, wobei die Vorrichtung

(a) eine Vorrichtung aufweist, die im Wesentlichen kontinuierlich und nicht-invasiv das Verhältnis κ der Blutströmungsgeschwindigkeit der Person zur Ausbreitungsgeschwindigkeit der Pulswelle der Person bestimmt,
(b) eine Vorrichtung (16) aufweist, die κ im Wesentlichen in Echtzeit verarbeitet, so daß die Momentanwerte der Größen SP, CO und VR der Person erhalten werden, und
(c) eine Vorrichtung aufweist, welche die Größen SP, CO und VR der Person in Echtzeit verarbeitet, um die Momentanwerte des algorithmischen Ausdrucks

$$\mathtt{INDEX2} = \partial SP/\partial CO - \partial SP/\partial VR$$

zu erhalten, wobei eine Zunahme von INDEX2 über die Zeit eine Änderung im Blutdruck der Person aufgrund einer Änderung des Herzminutenvolumens anzeigt oder sonst die Änderung im Blutdruck der Person auf einer Änderung im Gefäßwiderstand beruht.

**Revendications**

1. Procédé d'obtention de façon continue et non invasive d'un ou plusieurs paramètres d'un sujet à partir de la liste comprenant :

i. une pression sanguine systolique,
ii. une pression sanguine diastolique,
iii. un module de Young d'une artère,
iv. un débit cardiaque,
v. des changements relatifs de résistance vasculaire, et
vi. des changements relatifs d'élasticité vasculaire,

le procédé comprenant les étapes consistant à :

a) obtenir sensiblement de façon continue et non invasive le rapport, κ, de la vitesse d'écoulement sanguin du sujet à la vitesse de propagation de l'onde impulsionnelle du sujet,
b) traiter κ sensiblement en temps réel afin d'obtenir les valeurs instantanées des paramètres désirés.

2. Procédé selon la revendication 1, dans lequel κ est obtenu en traitant un signal de pléthysmographie PG et un temps de transit d'impulsion, PTT, de façon continue et non invasive, provenant du sujet.

**3.** Procédé selon la revendication 2, dans lequel $\kappa$ est obtenu selon l'expression algorithmique suivante :

$$\kappa \;=\; 1/\; (1/(PEAK.v)\;+\;1)$$

où v est la vitesses d'impulsion, et

$$PEAK \;=\; k_1.PTT.PA \;+\; k_2.AREA$$

où PA et AREA sont respectivement l'amplitude et la surface de l'onde impulsionnelle obtenues d'après le signal PG et $k_1$ et $k_2$ sont obtenus de façon empirique .

**4.** Procédé selon la revendication 2, dans lequel $\kappa$ est obtenu selon l'expression algorithmique suivante :

$$\kappa = \frac{1}{\left(\left(\dfrac{1}{PA}\right)+1\right)}$$

où PA est l'amplitude de l'onde impulsionnelle obtenue d'après le signal PG.

**5.** Procédé selon les revendications 3 et 4, comprenant en outre le fait d'éliminer par filtrage des fluctuations lentes dans l'onde impulsionnelle.

**6.** Procédé selon la revendication 5, dans lequel les fluctuations lentes dans PEAK sont éliminées par filtrage en remplaçant PEAK dans la revendication 3 par PEAK/(composante lente de PEAK)$^2$.

**7.** Procédé selon la revendication 1, dans lequel le traitement stipulé à l'étape a) pour le calcul de la pression sanguine systolique du sujet comprend l'expression algorithmique suivante :

$$SP \;=\; \rho v^2 \Phi(\kappa,\;\gamma)$$

où $\rho$ est la densité sanguine, $\gamma$ est l'exposant de Poisson thermodynamique du sang, v est la vitesse d'onde impulsionnelle et

$$\Phi = \frac{\sqrt{2\kappa(\gamma-1)^2 + 4\cdot(\gamma-1)+1}-1}{2(\gamma-1)}$$

**8.** Procédé selon la revendication 1, dans lequel le traitement stipulé dans l'étape a) pour le calcul de la pression sanguine systolique du sujet comprend l'expression algorithmique suivante :

$$SP \;=\; (\log\; v^2)/\alpha \;+\; 2\rho v^2 \kappa/3 \;+\; \lambda$$

où $\rho$ est la densité sanguine, v est la vitesse d'onde impulsionnelle, et $\lambda = (\log (2\rho R/E_0 h))/\alpha$, où R est le rayon de l'artère, $E_0$ est le module de Young en référence à la pression zéro, h est l'épaisseur de la paroi artérielle et $\alpha$ est obtenu de façon empirique.

9. Procédé selon la revendication 1, dans lequel le traitement stipulé à l'étape a) pour le calcul de la pression sanguine systolique du sujet comprend l'expression algorithmique

$$\text{SP = [(log v}^2\text{)/}\alpha\text{ + }\lambda\text{] / (1 - }\kappa\text{)}$$

où v est la vitesse d'onde impulsionnelle et $\lambda = (\log (2\rho R/E_0 h))/\alpha$, où R est le rayon de l'artère, $E_0$ est le module de Young référencé à la pression zéro, $\rho$ est la densité sanguine, h est l'épaisseur de la paroi artérielle et $\alpha$ est obtenu empiriquement.

10. Procédé selon la revendication 1, dans lequel le traitement stipulé à l'étape a) pour le calcul de la pression sanguine systolique du sujet comprend l'expression algorithmique

$$\text{SP = (log v}^2\text{/(1 - }\epsilon\text{H}^2\text{))/}\alpha\text{ + 2}\rho\text{v}^2\kappa\text{/3 + }\lambda$$

où $\rho$ est la densité sanguine, v est la vitesse d'onde impulsionnelle, $\lambda = (\log(2\rho R/E_0 h))/\alpha$, où R est le rayon de l'artère, $E_0$ est le module de Young référencé à la pression zéro, h est l'épaisseur de la paroi artérielle et $\epsilon$ et $\alpha$ sont obtenus de façon empirique.

11. Procédé selon la revendication 1, dans lequel le traitement stipulé à l'étape a) pour le calcul de la pression sanguine systolique du sujet comprend l'expression algorithmique

$$\text{SP = [(log v}^2\text{/(1 - }\epsilon\text{H}^2\text{))/}\alpha\text{ + }\lambda\text{] / (1 - }\kappa\text{)}$$

où v est la vitesse d'onde impulsionnelle, H est le rythme cardiaque, $\lambda = (\log(2\rho R/E_0 h))/\alpha$, où R est le rayon de l'artère, $E_0$ est le module de Young référencé à la pression zéro, h est l'épaisseur de la paroi artérielle, $\rho$ est la densité sanguine, et $\epsilon$ et $\alpha$ sont obtenus de façon empirique.

12. Procédé selon la revendication 1, dans lequel le traitement stipulé à l'étape a) pour le calcul de la pression sanguine diastolique du sujet comprend l'expression algorithmique

$$\text{DP = SP - }\rho\text{v}^2\kappa$$

où SP est la pression systolique, $\rho$ est la densité sanguine, et v est la vitesse d'onde impulsionnelle.

13. Procédé selon la revendication 1, dans lequel le traitement stipulé à l'étape a) pour le calcul du module de Young d'une artère du sujet comprend l'expression algorithmique:

$$\text{E = (2R/h) (SP - DP)/}\kappa$$

où R est le rayon de l'artère, h est l'épaisseur de la paroi artérielle, SP est la pression systolique et DP est la pression diastolique.

14. Procédé selon la revendication 1, dans lequel le traitement stipulé à l'étape a) pour le calcul du module de Young d'une artère du sujet comprend l'expression algorithmique :

$$\text{E = (2R/h) SP / }\Phi\text{(}\kappa\text{, }\gamma\text{)}$$

$$\Phi = \frac{\sqrt{2\kappa(\gamma-1)^2 + 4 \cdot (\gamma-1) + 1} - 1}{2(\gamma-1)}$$

**15.** Procédé selon la revendication 1, dans lequel le calcul stipulé à l'étape a) pour le calcul du module de Young d'une artère du sujet comprend l'expression algorithmique :

$$E = (2R/h) \ \rho \ \exp \ (-\lambda + MP)\alpha$$

où R est le rayon de l'artère, h est l'épaisseur de la paroi artérielle, SP est la pression systolique $\gamma$ est l'exposant de Poisson thermodynamique du sang et

où R est le rayon de l'artère, h est l'épaisseur de la paroi artérielle, $\rho$ est la densité sanguine, MP = (SP + 2.DP)/3 où SP est la pression systolique, DP est la pression diastolique et où au moins l'une parmi la pression systolique et la pression diastolique est obtenue en utilisant une expression algorithmique impliquant $\kappa$, et $\lambda$ = (log (2$\rho$R/E$_0$h)) /$\alpha$, où $E_0$ est le module de Young référencé à la pression zéro, et $\alpha$ est une constante obtenue de façon empirique.

**16.** Procédé selon la revendication 1, dans lequel le traitement stipulé à l'étape a) pour le calcul du module de Young d'une artère du sujet comprend l'expression algorithmique :

$$E = (2R/h) \ \rho \ \exp \ ((-\lambda + SP \ (1 - \kappa))\alpha)$$

où R est le rayon de l'artère, h est l'épaisseur de la paroi artérielle, $\rho$ est la densité sanguine, SP est la pression systolique et $\lambda$ = (log(2$\rho$R/E$_0$h))/$\alpha$, où $E_0$ est le module de Young référencé à la pression zéro, et $\alpha$ est une constante obtenue de façon empirique.

**17.** Procédé selon la revendication 1, dans lequel le traitement stipulé à l'étape a) pour le calcul du changement relatif du débit cardiaque d'un sujet comprend l'expression algorithmique :

$$CO = PEAK \ \{v \ [1 + SP/(2\rho v^2)]\}^2$$

où SP est une pression systolique obtenue en utilisant une expression algorithmique impliquant $\kappa$, $\rho$ est la densité sanguine, et v est la vitesse d'onde impulsionnelle et

$$PEAK = k_1 \ . \ PTT.PA + k_2 \ . \ AREA$$

où PA et AREA sont respectivement l'amplitude et la surface de la crête d'onde impulsionnelle d'un signal PG, et $k_1$ et $k_2$ sont obtenus de façon empirique.

**18.** Procédé selon la revendication 14 comprenant en outre le filtrage d'élimination de fluctuations lentes d'onde impulsionnelle.

**19.** Procédé selon la revendication 1, dans lequel le traitement stipulé à l'étape a) pour le calcul de la résistance cardiaque du sujet comprend l'expression algorithmique :

$$VR = (SP - DP) \ / \ CO$$

où l'un ou plusieurs parmi SP, DP et CO est obtenu par un calcul impliquant $\kappa$.

20. Procédé selon la revendication 1, dans lequel le traitement stipulé à l'étape a) pour le calcul du changement relatif de l'élasticité vasculaire d'un sujet comprend l'expression algorithmique

$$VC = PEAK / (SP - DP)$$

où

$$PEAK = k_1 \ . \ PTT.PA + k_2 \ . \ AREA$$

où PA et AREA sont respectivement l'amplitude et la surface de l'onde impulsionnelle obtenue d'après le signal PG, et $k_1$ et $k_2$ sont obtenus empiriquement.

21. Procédé pour déterminer de façon continue et non invasive si un changement de la pression sanguine d'un sujet est dû à un changement du débit cardiaque ou d'élasticité vasculaire, comprenant le fait de :

a) obtenir sensiblement de façon continue et non invasive le rapport, $\kappa$, de la vitesse d'écoulement sanguin du sujet à la vitesse de propagation d'onde impulsionnelle du sujet,
b) traiter $\kappa$ sensiblement en temps réel pour ainsi obtenir les valeurs instantanées de SP, CO et VC du sujet, et
c) traiter les SP, CO et VC du sujet en temps réel afin d'obtenir les valeurs instantanées de l'expression algorithmique :

$$INDEX1 = \partial SP/\partial CO - \partial SP/\partial VC$$

un accroissement de INDEX1 au fil du temps indiquant un changement de la pression sanguine du sujet dû à un changement du débit cardiaque, sinon le changement de la pression sanguine du sujet est dû à un changement d'élasticité vasculaire.

22. Procédé pour déterminer de façon continue et non invasive si un changement de la pression sanguine d'un sujet est due à un changement du débit cardiaque du sujet ou de résistance vasculaire, comprenant le fait de :

a) obtenir sensiblement de façon continue et non invasive le rapport, $\kappa$, de la vitesse d'écoulement sanguin du sujet à la vitesse de propagation d'onde impulsionnelle du sujet,
b) traiter $\kappa$ sensiblement en temps réel afin d'obtenir les valeurs instantanées de SP, CO et VR du sujet, et
c) traiter les SP, CO et VR du sujet en temps réel afin d'obtenir les valeurs instantanées de l'expression algorithmique :

$$INDEX2 = \partial SP/\partial CO - \partial SP/\partial VR$$

un accroissement de INDEX2 au fil du temps indiquant un changement de la pression sanguine du sujet dû à un changement du débit cardiaque, sinon le changement de la pression sanguine du sujet est dû à un changement de résistance vasculaire.

23. Dispositif d'obtention de façon continue et non invasive d'un ou plusieurs des paramètres vasculaires d'un sujet (10) à partir de la liste comprenant :

i. une pression sanguine systolique
ii. une pression sanguine diastolique
iii. un module de Young d'une artère

iv. un changement relatif de débit cardiaque

v. un changement relatif de résistance vasculaire, et

vi. un changement relatif d'élasticité vasculaire,

le dispositif comprenant :

a) un dispositif d'obtention sensiblement de façon continue et non invasive du rapport $\kappa$ de la vitesse d'écoulement sanguin du sujet à la vitesse de propagation d'onde impulsionnelle du sujet, et

b) un dispositif (16) traitant $\kappa$ sensiblement en temps réel afin d'obtenir les valeurs instantanées des paramètres désirés.

24. Dispositif selon la revendication 23, dans lequel $\kappa$ est obtenu par traitement d'un pléthysmographe PG et d'un temps de transit d'impulsion PTT de façon continue et non invasive obtenus à partir du sujet (10).

25. Dispositif selon la revendication 24, dans lequel $\kappa$ est obtenu selon l'expression algorithmique suivante :

$$\kappa = 1/(1/(\text{PEAK.v}) + 1)$$

où v est inversement proportionnel à PTT, et

$$\text{PEAK} = k_1 \cdot \text{PTT.PA} + k_2 \cdot \text{AREA}$$

où PA et AREA sont respectivement l'amplitude et la surface de l'onde impulsionnelle obtenue à partir du signal PG et $k_1$ et $k_2$ sont obtenus de façon empirique.

26. Dispositif selon la revendication 23, dans lequel $\kappa$ est obtenu selon l'expression algorithmique suivante :

$$\kappa = 1/((1/\text{PA}) + 1)$$

où PA est l'amplitude de l'onde impulsionnelle obtenue à partir du signal PG.

27. Dispositif selon la revendication 25 apte à éliminer par filtrage des fluctuations lentes de l'onde impulsionnelle.

28. Dispositif selon la revendication 27, dans lequel les fluctuations lentes de l'onde impulsionnelle sont éliminées par filtrage en remplaçant PEAK dans la revendication 22 par PEAK/ (composante lente de PEAK)$^2$.

29. Dispositif selon la revendication 23, dans lequel le traitement stipulé à l'étape a) pour le calcul de la pression sanguine systolique du sujet comprend l'expression algorithmique :

$$\text{SP} = \rho v^2 \Phi(\kappa, \gamma)$$

où $\rho$ est la densité sanguine, $\gamma$ est l'exposant de Poisson thermodynamique du sang, v est la vitesse d'onde impulsionnelle et

$$\Phi = \frac{\sqrt{2\kappa(\gamma-1)^2 + 4 \cdot (\gamma-1) + 1} - 1}{2(\gamma-1)}$$

30. Dispositif selon la revendication 23, dans lequel le traitement stipulé à l'étape a) pour le calcul de la pression sanguine systolique du sujet comprend l'expression algorithmique :

$$SP = (\log v^2)/\alpha + 2\rho v^2 \kappa/3 + \lambda$$

où $\rho$ est la densité sanguine et $\lambda = (\log 2\rho R/E_0 h)/\alpha$, où R est le rayon de l'artère, $E_0$ est le module de Young référencé à la pression zéro, h est l'épaisseur de la paroi artérielle et $\alpha$ est obtenu de façon empirique.

**31.** Dispositif selon la revendication 23, dans lequel le traitement stipulé à l'étape a) pour le calcul de la pression sanguine systolique du sujet comprend l'expression algorithmique :

$$SP = [(\log v^2)/\alpha + \lambda] / (1 - \kappa)$$

où v est la vitesse d'onde impulsionnelle et $\lambda = (\log (2\rho R/E_0 h))/\alpha$, où R est le rayon de l'artère, $E_0$ est le module de Young référencé à la pression zéro, $\rho$ est la densité sanguine, h est l'épaisseur de la paroi artérielle et $\alpha$ est obtenu de façon empirique.

**32.** Dispositif selon la revendication 23, dans lequel le traitement stipulé à l'étape a) pour le calcul de la pression sanguine systolique du sujet comprend l'expression algorithmique :

$$SP = (\log v^2/(1 - \epsilon H^2))/\alpha + 2\rho v^2 \kappa/3 + \lambda$$

où $\rho$ est la densité sanguine, v est la vitesse d'onde impulsionnelle, H est le rythme cardiaque, $\lambda = (\log (2\rho R/E_0 h))/\alpha$, où R est le rayon de l'artère, $E_0$ est le module de Young référencé à la pression zéro, h est l'épaisseur de la paroi artérielle et $\epsilon$ et $\alpha$ sont obtenus de façon empirique.

**33.** dispositif selon la revendication 23, dans lequel le traitement stipulé à l'étape a) pour le calcul de la pression sanguine systolique du sujet comprend l'expression algorithmique :

$$SP = [(\log v^2/(1 - \epsilon H^2))/\alpha + \lambda] / (1 - \kappa)$$

où v est la vitesse d'onde impulsionnelle, H est le rythme cardiaque, $\lambda = (\log (2\rho R/E_0 h))/\alpha$, où R est le rayon de l'artère, $E_0$ est le module de Young référencé à la pression zéro, h est l'épaisseur de la paroi artérielle, $\rho$ est la densité sanguine, et $\epsilon$ et $\alpha$ sont obtenus de façon empirique.

**34.** Dispositif selon la revendication 23, dans lequel le traitement stipulé à l'étape a) pour le calcul de la pression sanguine diastolique du sujet comprend l'expression algorithmique :

$$DP = SP - \rho v^2 \kappa$$

où SP est la pression systolique, $\rho$ est la densité sanguine, et v est la vitesse d'onde impulsionnelle.

**35.** Dispositif selon la revendication 23, dans lequel le traitement stipulé à l'étape a) pour le calcul du module de Young d'une artère du sujet comprend l'expression algorithmique :

$$E = (2R/h) (SP - DP)/\kappa$$

où R est le rayon de l'artère, h est l'épaisseur de la paroi artérielle, SP est la pression systolique et DP est la pression diastolique.

**36.** Dispositif selon la revendication 23, dans lequel le traitement stipulé à l'étape a) pour le calcul du module de Young d'une artère du sujet comprend l'expression algorithmique :

$$E = (2R/h) \ SP/\Phi(\kappa, \ \gamma)$$

où R est le rayon de l'artère, h est l'épaisseur de la paroi artérielle, SP est la pression systolique, $\gamma$ est l'exposant de Poisson thermodynamique du sang et

$$\Phi = \frac{\sqrt{2\kappa(\gamma-1)^2 + 4\cdot(\gamma-1)+1} - 1}{2(\gamma-1)}$$

**37.** Dispositif selon la revendication 23, dans lequel le traitement stipulé à l'étape a) pour le calcul du module de Young d'une artère du sujet comprend l'expression algorithmique :

$$E = (2R/h) \ \rho \ \exp \ (-\lambda + MP)\alpha$$

où R est le rayon de l'artère, h est l'épaisseur de la paroi artérielle, $\rho$ est la densité sanguine, MP = (SP + 2 DP) /3 où SP est la pression systolique, DP est la pression diastolique, où au moins l'une parmi la pression systolique et la pression diastolique est obtenue en utilisant une expression algorithmique impliquant $\kappa$, et $\lambda$ = (log (2$\rho$R/E$_0$h))/$\alpha$ où E$_0$ est le module de Young référencé à la pression zéro et $\alpha$ est une constante obtenue de façon empirique.

**38.** Dispositif selon la revendication 35, dans lequel le traitement stipulé à l'étape a) pour le calcul du module de Young d'une artère du sujet comprend l'expression algorithmique :

$$E = (2R/h) \ \rho \ \exp \ ((-\lambda + SP(1 - \kappa))\alpha$$

où R est le rayon de l'artère, h est l'épaisseur de la paroi artérielle, $\rho$ est la densité sanguine, SP est la pression systolique et $\lambda$ = (log (2$\rho$R/E$_0$h))/$\alpha$, où E$_0$ est le module de Young référencé à la pression zéro et $\alpha$ est une constante obtenue de façon empirique.

**39.** Dispositif selon la revendication 23, dans lequel le traitement stipulé à l'étape a) pour le calcul du changement relatif du débit cardiaque d'un sujet comprend l'expression algorithmique :

$$CO = PEAK \ \{v \ [1 + SP/ \ (2\rho \ v^2)] \}^2$$

où SP est une pression systolique obtenue en utilisant une expression algorithmique impliquant $\kappa$ et

$$PEAK = k_1.PTT.PA + k_2.AREA$$

où PA et AREA sont respectivement l'amplitude et la surface de l'onde impulsionnelle obtenues d'après un signal PG, et $k_1$ et $k_2$ sont obtenus de façon empirique.

**40.** Dispositif selon la revendication 23, dans lequel le traitement stipulé à l'étape a) pour le calcul de la résistance cardiaque du sujet comprend l'expression algorithmique :

$$VR = (SP - DP) / CO$$

où l'un quelconque parmi SP, DP et CO est obtenu d'après un calcul impliquant κ.

**41.** Dispositif déterminant de façon contenue et non invasive si un changement de la pression sanguine d'un sujet est dû à un changement du débit cardiaque ou de l'élasticité vasculaire, comprenant :

a) un dispositif obtenant sensiblement de façon continue et non invasive le rapport κ de la vitesse d'écoulement sanguin du sujet à la vitesse de propagation d'onde impulsionnelle du sujet,
b) un dispositif (16) traitant κ sensiblement en temps réel afin d'obtenir les valeurs instantanées des SP, CO et VC du sujet, et
c) un dispositif traitant les SP, CO et VC du sujet en temps réel afin d'obtenir les valeurs instantanées de l'expression algorithmique :

$$INDEX1 = \partial SP / \partial CO - \partial SP / \partial VC$$

un accroissement de INDEX1 au fil du temps indiquant un changement de la pression sanguine du sujet dû à un changement du débit cardiaque, sinon le changement de la pression sanguine du sujet est dû à un changement de souplesse vasculaire.

**42.** Dispositif déterminant de façon continue et non invasive si un changement de la pression sanguine d'un sujet est dû à un changement de la résistance vasculaire du sujet comprenant :

a) un dispositif obtenant sensiblement de façon continue et non invasive le rapport κ de la vitesse d'écoulement sanguin du sujet à la vitesse de propagation d'onde impulsionnelle du sujet,
b) un dispositif (16) traitant κ sensiblement en temps réel afin d'obtenir les valeurs instantanées des SP, CO et VR du sujet, et
c) un dispositif traitant les SP, CO et VR du sujet en temps réel afin d'obtenir les valeurs instantanées de l'expression algorithmique :

$$INDEX2 = \partial SP/\partial CO - \partial SP/\partial VR$$

un accroissement de INDEX2 au fil du temps indiquant un changement de la pression sanguine du sujet dû à un changement du débit cardiaque, sinon le changement de la pression sanguine du sujet est dû à un changement de résistance vasculaire.

FIG.1

EP 1 150 604 B1

```
┌─────────────────────┐
│   OBTAIN THE ECG    │
│   AND PC SIGNALS    │
└─────────────────────┘
           │
┌─────────────────────┐
│     CALCULATE       │
│        K            │
└─────────────────────┘
           │
┌─────────────────────┐
│  CALCULATE FROM     │
│  K THE PARAMETERS   │
│    OF INTEREST      │
└─────────────────────┘
           │
┌─────────────────────┐
│    TRANSFER THE     │
│   RESULTS OF THE    │
│  CALCULATIONS TO PC │
│  FOR STORAGE AND/OR │
│      DISPLAY        │
└─────────────────────┘
```

FIG.2

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 4475554 A
- US 4869262 A
- US 4807638 A
- US 5709212 A

- EP 443267 A
- US 5309916 A
- EP 443267 A1

### Non-patent literature cited in the description

- **LANDAU, L.D. ; LIFSHITZ E.M.** Statistical Physics. Pergamon Press, 1979
- **LANDAU, L.D. ; LIFSHITZ E.M.** Fluid Mechanics. Pergamon Press, 1987

- **LANDAU, L.D. ; LIFSHITZ E.M.** Theory of Elasticity. Pergamon Press, 1986
- **KAPLAN D ; GLASS.** Understanding Non-Linear Dynamics. Springer-Verlag, 1995